(19) Europäisches Patentamt
European Patent Office
Office européen des brevets

(11) Veröffentlichungsnummer: **0 343 110**
**A1**

(12) # EUROPÄISCHE PATENTANMELDUNG

(21) Anmeldenummer: **89810346.0**

(22) Anmeldetag: **11.05.89**

(51) Int. Cl.[4]: **C 07 C 127/22**
A 01 N 47/34

(30) Priorität: **20.05.88 CH 1921/88**

(43) Veröffentlichungstag der Anmeldung:
**23.11.89 Patentblatt 89/47**

(84) Benannte Vertragsstaaten:
**BE CH DE FR GB IT LI NL**

(71) Anmelder: **CIBA-GEIGY AG**
**Klybeckstrasse 141**
**CH-4002 Basel (CH)**

(72) Erfinder: **Drabek, Jozef, Dr.**
**Benkenstrasse 12**
**CH-4104 Oberwil (CH)**

(54) **Benzoylphenylharnstoffe.**

(57) Neue substituierte N-(2,4,6-Trifluorbenzoyl)-N'-(2,5-dihalogen-4-halogenalkoxy-phenyl)-harnstoffe der Formel I

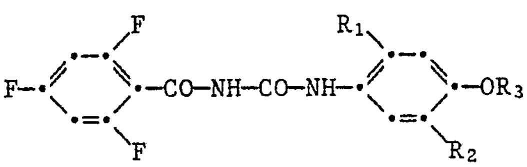 (I),

worin
$R_1$ für Fluor oder Chlor;
$R_2$ für Fluor, Chlor oder Brom; und
$R_3$ für ein- oder mehrfach durch Halogen substituiertes $C_1$-$C_7$-Alkyl
stehen, Verfahren zu ihrer Herstellung, ihre Verwendung in der Schädlingsbekämpfung und Schädlingsbekämpfungsmittel, welche mindestens eine Verbindung der Formel I als Wirkstoff enthalten, werden offenbart. Bevorzugtes Anwendungsgebiet ist die Kontrolle von Schädlingen an Tieren und Pflanzen.

EP 0 343 110 A1

**Beschreibung**

## Benzoylphenylharnstoffe

Die vorliegende Erfindung betrifft neue substituierte N-(2,4,6-Trifluorbenzoyl)-N′-(2,5-dihalogen-4-halogenalkoxy-phenyl)-harnstoffe, Verahren zu ihrer Herstellung, Schädlingsbekämpfungsmittel, die diese Verbindungen enthalten, sowie ihre Verwendung bei der Kontrolle von Schädlingen.

Die erfindungsgemässen Verbindungen entsprechen der Formel I

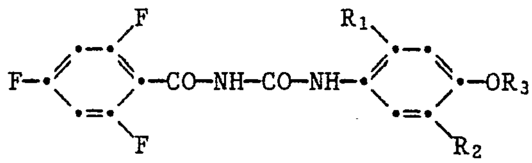

(I),

worin
$R_1$ für Fluor oder Chlor;
$R_2$ für Fluor, Chlor oder Brom; und
$R_3$ für ein- oder mehrfach durch Halogen substituiertes $C_1$-$C_7$-Alkyl
stehen.

Die als Substituenten in Betracht kommenden ein- oder mehrfach durch Halogen substituierten $C_1$-$C_7$-Alkyl können geradkettig oder verzweigt und nur teilweise oder auch perhalogeniert sein, wobei als Halogene sowohl Fluor und Chlor als auch Brom und Jod, wobei Fluor und Chlor bevorzugt sind, und als Alkyle Methyl, Aethyl, Propyl, i-Propyl, Butyl, i-Butyl, sek.Butyl, tert.Butyl oder Pentyl, Hexyl oder Heptyl und ihre Isomeren genannt seien. Geeignete Beispiele solcher Substituenten sind u.a. das ein- bis dreifach durch Fluor, Chlor und/oder Brom substituierte Methyl wie z.B. $CHF_2$ oder $CF_3$; das ein- bis fünffach durch Fluor, Chlor und/oder Brom substituierte Aethyl wie z.B. $CHFCF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CH_3$, $CF_2CHBrF$ oder $CClFCHClF$; das ein- bis siebenfach durch Fluor, Chlor und/oder Brom substituierte Propyl oder Isopropyl wie z.B. $CF_2CHBrCH_2Br$, $CF_2CHFCF_3$, $CH_2CF_2CF_3$ oder $CF_2CF_2CF_3$; das ein- bis neunfach durch Fluor, Chlor und/oder Brom substituierte Butyl oder eines seiner Isomeren wie z.B. $CF_2CF_2CHFCF_3$, $(CF_2)_3CF_3$, $CF_2CHFCF_2CF_3$ oder $CH_2(CF_2)_2CF_3$. Sofern der Halogenalkylrest asymmetrische Kohlenstoffatome enthält, stellen die Verbindungen der Formel I Enantiomerenpaare dar. Im Rahmen der Erfindung sind unter Formel I sowohl die reinen Enantiomere als auch die Racemate zu verstehen.

Unter den Verbindungen der Formel I stehen diejenigen im Vordergrund, in denen $R_3$ für ein- oder mehrfach durch Fluor und/oder Chlor substituiertes $C_1$-$C_5$-Alkyl stehen.

Dabei sind diejenigen Verbindungen der Formel I bevorzugt, in denen $R_1$, und $R_2$ je für Fluor oder Chlor; und $R_3$ für ein- oder mehrfach durch Fluor substituiertes $C_1$-$C_4$-Alkyl stehen.

Die erfindungsgemässen Verbindungen können nach im Prinzip bekannten Verfahren hergestellt werden. Solche Verfahren sind u.a. in den DE-OS 21 23 236, 26 01 780 und 32 40 975 beschrieben. So können die Verbindungen der Formel I z.B. erhalten werden, indem man

a) ein Anilin der Formel II

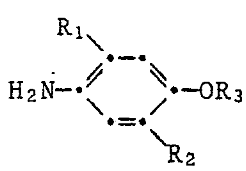

(II)

mit einem Benzoylisocyanat der Formel III

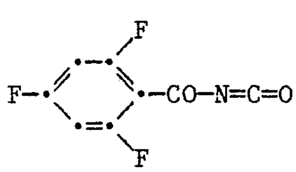

(III),

oder

b) ein Isocyanat der Formel IV

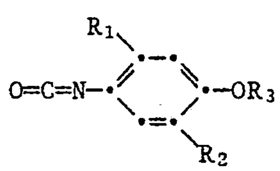

(IV)

mit einem Benzamid der Formel V

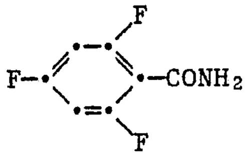

(V),

oder

c) ein Anilin der Formel II mit einem Urethan der Formel VI

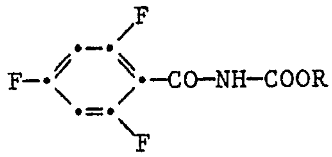

(VI)

umsetzt. In den Formeln III bis VI haben $R_1$, $R_2$ und $R_3$ die für Formel I angegebene Bedeutung und in Formel VI steht R für einen $C_1$-$C_8$-Alkylrest, der gegebenenfalls mit Halogen, vorzugsweise Chlor, substituiert sein kann.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylole, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis +200°C, vorzugsweise zwischen 0 und +100°C, z.B. bei Raumtemperatur, gegebenenfalls in Gegenwart einer organischen Base, z.B. Triäthylamin, durchgeführt. Die Durchführung von Verfahren b) erfolgt bei einer Temperatur von 0 bis +150°C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für das Verfahren c), d.h. für die Umsetzung der Urethane der Formel VI mit dem Anilin der Formel II, werden Temperaturen zwischen etwa +60°C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln II bis VI sind teilweise bekannt. Sowohl die bekannten als auch die gegebenenfalls neuen Ausgangsstoffe können analog bekannten Verfahren hergestellt werden.

In der US Patentschrift 4,518,804 werden ganz allgemein u.a. N-Halogenphenyl-N′-(2,5-dihalogen-4-halogenalkoxy)-phenylharnstoffe als Insektizide mit larvizider Wirkung beschrieben. In dieser Veröffentlichung werden jedoch keine N-(2,4,6-Trifluorbenzoyl)-N′-(2,5-dihalogen-4-halogenalkoxyphenyl)-harnstoffe spezifisch offenbart.

Ueberraschenderweise wurde gefunden, dass die erfindungsgemässen Verbindungen der Formel I bei günstiger Warmblüter- und Pflanzenverträglichkeit stärkere Wirkstoffe in der Schädlingsbekämpfung sind. So eignen sich die Verbindungen der Formel I z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formel I von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus- und Nutztieren, kommen vor allem Ektoparasiten, wie z.B. Milben und Zecken und Dipteren, wie z.B. Lucilia sericata in Betracht. Weiter wurde gefunden, dass die erfindungsgemässen Wirkstoffe repellierend und frassabschreckend gegen landlebende Nackt-und Gehäuseschnecken wirken und somit zum Schutz von Kultur- und Zierpflanzen vor Schäden durch Schneckenfrass verwendet werden können.

Insbesondere zeichnen sich die erfindungsgemässen Verbindungen durch eine ausgezeichnete larvizide Wirkung bei Spodoptera littoralis und Heliothis virescens aus.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände

anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen, inerten, pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die einen Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze

liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung

1.1. N-(2,4,6-Trifluorbenzoyl)-N′-[2,5-dichlor-4-(1′,1′,2′,3′,3′,3′-hexafluorpropoxy)-phenyl]-harnstoff

4,6 g 2,5-dichlor-4-(1′,1′,2′,3′,3′,3′-hexafluorpropoxy)-anilin, gelöst in 50 ml trockenem Toluol, werden bei Raumtemperatur unter Feuchtigkeitsausschluss mit 2,8 g 2,4,6-Trifluorbenzoylisocyanat, gelöst in 5 ml trockenem Toluol versetzt und 10 Stunden lang gerührt. Anschliessend werden etwa 75 % des Lösungsmittels am Rotationsverdampfer entfernt. Der entstandene Niederschlag wird abgenutscht, mit wenig kaltem Hexan gewaschen und im Vakuum getrocknet. Die Titelverbindung der Formel

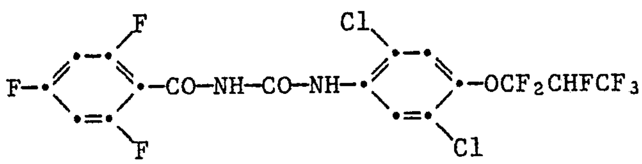

(Verb. Nr. 1.1.)

liegt als farbloses kristallines Pulver vor; Smp. 193-194°C.

Auf analoge Weise werden die folgenden Verbindungen hergestellt:

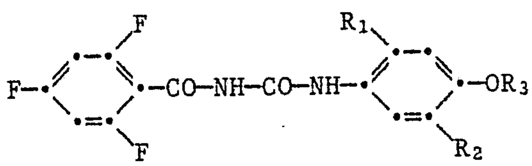

| Verb. Nr. | $R_1$ | $R_2$ | $R_3$ | phys. Daten |
|---|---|---|---|---|
| 1.2. | F | F | $CF_2CHFCF_3$ | Smp. 185-187°C |
| 1.3. | F | Cl | $CF_2CHFCF_3$ | Smp. 172-173°C |
| 1.4 | Cl | Cl | $CF_2CHF_2$ | Smp. 200-202°C |
| 1.5 | Cl | Cl | $(CF_2)_2CF_3$ | Smp. 173-176°C |
| 1.6 | F | Cl | $CH_2CF_2CF_3$ | Smp. 160-162°C |
| 1.7 | Cl | Cl | $CF_2CHFBr$ | Smp. 206-208°C |
| 1.8 | F | Cl | $CH_2(CF_2)_5CF_3$ | Smp. 168-171°C |
| | Cl | Cl | $(CF_2)_6CF_3$ | |
| | Cl | Cl | $CF_2CHFCl$ | |
| | F | Br | $CF_2CHFCF_3$ | |
| | F | F | $CH_2(CF_2)_2CF_3$ | |
| | Cl | Cl | $CH_3$ | |
| | Cl | Cl | $CHF_2$ | |

Beispiel 2: Formulierungen von Wirkstoffen der Formel I gemäss Herstellungsbeispiel 1.
(% = Gewichtsprozent)

2.1. Emulsions-Konzentrate

| | a) | b) |
|---|---|---|
| Wirkstoff gemäss dem Herstellungsbeispiel 1. | 5 % | 12 % |
| K-Dodecylbenzolsulfonat | 4 % | 3 % |
| Ricinusöl-polyäthylenglykoläther (36 Mol AeO) | 6 % | 12 % |
| Cyclohexanon | 20 % | - |
| Xylolgemisch | 65 % | 48 % |
| N-Methyl-2-pyrrolidon | - | 20 % |
| Dimethylcyclohexylphthalat | - | 5 % |

Auch solchen Konzentraten können durch Verdünnen mit Wasser Emulsionen jeder gewünschten Konzentration hergestellt werden.

2.2. Lösungen

| | a) | b) |
|---|---|---|
| Wirkstoff gemäss dem Herstellungsbeispiel 1. | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190° C) | - | 74 % |

Die Lösungen sind zur Anwendung in Form kleinster Tropfen geeignet.

2.3. Granulate

| | a) | b) |
|---|---|---|
| Wirkstoff gemäss dem Herstellungsbeispiel 1. | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4. Extruder Granulat

| | |
|---|---|
| Wirkstoff gemäss dem Herstellungsbeispiel 1. | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5. Umhüllungs-Granulat

| | |
|---|---|
| Wirkstoff gemäss dem Herstellungsbeispiel 1. | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

2.6. Stäubemittel

| | a) | b) | c) | d) |
|---|---|---|---|---|
| Wirkstoff gemäss dem Herstellungsbeispiel 1. | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

2.7. Spritzpulver

| | a) | b) | c) |
|---|---|---|---|
| Wirkstoff gemäss dem Herstellungsbeispiel 1. | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

2.8. Suspensions-Konzentrat

| | |
|---|---|
| Wirkstoff gemäss dem Herstellungsbeispiel 1. | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologische Prüfungen

3.1. Wirkung gegen Schmeissfliegen

Frisch abgelegte Eier der Schmeissfliegenart Lucilia sericata werden in kleinen Portionen (30-50 Eier) in Reagenzgläser gegeben, in denen zuvor 4 ml Nährmedium mit 1 ml Testlösung, die 80 ppm des zu prüfenden Wirkstoffes enthält, vermischt worden sind. Nach Beimpfung des Kulturmediums werden die Testgläser mit einem Wattestopfen verschlossen und im Brutschrank bei 30°C 4 Tage lang bebrütet. Im unbehandelten Medium entwickeln sich bis zu diesem Zeitpunkt ca. 1 cm lange Larven (Stadium 3). Ist die Substanz aktiv, so

sind die Larven zu diesem Zeitpunkt entweder tot oder deutlich zurückgeblieben. Die Auswertung erfolgt nach 96 Stunden; es wird die Mortalität in Prozenten bestimmt.

Verbindungen gemäss Beispiel 1 zeigen gute larvizide Wirkung.

3.2. Wirkung gegen Aedes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 12,5 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss Beispiel 1. zeigen gute Wirkung in diesem Test.

3.3. Frassgift-Wirkung auf Spodoptera littoralis-Larven ($L_1$)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvalen Stadium besetzt. Der Versuch wird bei 26°C und ca. 50 % relativer Luftfeuchtigkeit durchgeführt. Nach 2 und 3 Tagen wird die Mortalität und nach 5 Tagen werden Entwicklungs- und Häutungsstörungen der angesetzten Larven bestimmt.

Verbindungen gemäss Beispiel 1. zeigen 100%ige Wirkung.

3.4. Frassgift-Wirkung auf Spodoptera littoralis- und Heliothis virescens-Larven ($L_3$)

Eingetopfte Sojapflanzen (Topfgrösse 10 cm Durchmesser) im 4-Blatt-Stadium werden mit wässrigen Wirkstoffemulsionen besprüht, die den Wirkstoff in einer Konzentration von 0,75 ppm enthalten.

Nach zwei Tagen werden die behandelten Sojapflanzen mit je 10 Larven von Spodoptera littoralis und Heliothis virescens im dritten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss Beispiel 1. zeigen bei Spodoptera und bei Heliothis nach 2 und 5 Tagen 80-100%ige Wirkung (Mortalität).

3.5. Frassfigt-Wirkung auf Plutella xylostella-Larven ($L_2$)

Eingetopfte Chinakohlpflanzen (Topfgrösse 10 cm Durchmesser) im 4-Blatt-Stadium werden mit einer wässrigen Wirkstoffemulsion besprüht, die den Wirkstoff in einer Konzentration von 0,75 ppm enthält.

Nach zwei Tagen werden die behandelten Chinakohlpflanzen mit 10 Plutella xylostella-Larven im zweiten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss Beispiel 1. zeigen nach 2 und 5 Tagen 80-100%ige Wirkung (Mortalität).

3.6. Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 400 ppm des zu prüfenden Wirkstoffes, eingetaucht. Nach dem Eintrocknen der Lösung wird das Filterpapier mit den Eiern in einer Petrischale ausgelegt und bei einer Temperatur von 28°C belassen. Nach 6 Tagen wird der prozentuale Schlupf aus den behandelten Eiern bewertet.

Verbindungen gemäss Beispiel 1. zeigen gute Wirkung in obigem Test.

3.7. Reproduktions-Beeinflussung von Anthonomus grandis

Baumwollpflanzen im Keimblattstadium werden mit einer wässrigen Dispersion der Testsubstanz (Konzentration 400 ppm) besprüht. Nach dem Antrocknen des Belags werden die Pflanzen mit je 10 adulten Anthonomus grandis besiedelt. Die Pflanzen werden mit Kunststoffzylindern mit Gazedeckeln abgedeckt. Nach 5 Tagen werden die Testtiere von den Pflanzen zur Eiablage auf kugelige Körper, bestehend aus einer künstlichen Diät ("Pellets"), in Kunststoffbecher überführt. Nach weiteren 5 Tagen werden die Käfer entfernt und die Pellets für 5 bis 7 Tage bei 26°C und 55 % relativer Luftfeuchtigkeit inkubiert. Anschiessend wird der Versuch ausgewertet, wobei die Anzahl abgelegter Eier und daraus geschlüpfter Larven mit der unbehandelten Kontrolle verglichen werden.

Verbindungen gemäss Beispiel 1 zeigen in obigem Test eine gute Wirkung gegen Anthonomus grandis.

3.8. Wirkung gegen Epilachna varivestis

Etwa 15-20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28°C und 60 % relativer Luftfeuchtigkeit durchgeführt.

Nach 2 und 3 Tagen wird die Mortalität in Prozenten bestimmt. Zur Auswertung hinsichtlich allfälligem Frassschaden (Antifeeding-Effekt), Entwicklungs- und Häutungsstörungen werden die Versuchstiere während weiterer 3 Tage beobachtet.

Verbindungen gemäss Beispiel 1. zeigen gute Wirkung im obigen Test.

3.9. Ovizide Wirkung auf Heliothis virescens und Spodoptera littoralis

Entsprechende Mengenanteile einer benetzbaren pulverförmigen Formulierung, enthaltend 25 Gew.% des zu prüfenden Wirkstoffes, werden mit jeweils soviel Wasser vermischt, dass sich eine wässrige Emulsion mit einer Wirkstoffkonzentration von 400 ppm ergibt.

In diese wirkstoffhaltige Emulsion werde eintägige auf Cellophan abgelegte Eigelege von Heliothis und auf Papier abgelegte Eigelege von Spodoptera während drei Minuten eingetaucht und dann auf Rundfilter abgenutscht. Die so behandelten Gelege werden in Petrischalen ausgelegt und bei 28° C und 60 % relativer Luftfeuchtigkeit in der Dunkelheit aufbewahrt. Nach 5 bis 8 Tagen wird die Schlupfrate, d.h. die Anzahl Larven, die sich aus den behandelten Eiern entwickelt haben, gegenüber unbehandelten Kontrollansätzen bestimmt.

Verbindungen gemäss Beispiel 1. zeigen in obigem Test eine 80-100%ige ovizide Wirkung (Mortalität) gegen Heliothis virescens und Spodoptera littoralis.

**Patentansprüche**

1. Verbindungen der Formel I

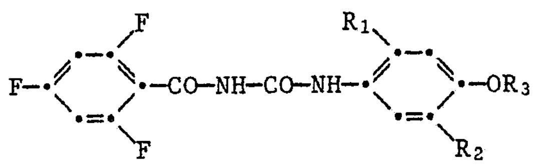 (I),

worin
$R_1$ für Fluor oder Chlor;
$R_2$ für Fluor, Chlor oder Brom; und
$R_3$ für ein- oder mehrfach durch Halogen substituiertes $C_1$-$C_7$-Alkyl stehen.

2. Verbindungen der Formel I gemäss Anspruch 1, worin $R_3$ für ein- oder mehrfach durch Fluor und/oder Chlor substituiertes $C_1$-$C_5$-Alkyl stehen.

3. Verbindungen der Formel I gemäss Anspruch 2, worin $R_1$ und $R_2$ je für Fluor oder Chlor; und $R_3$ für ein- oder mehrfach durch Fluor substituiertes $C_1$-$C_4$-Alkyl stehen.

4. Die Verbindungen gemäss Anspruch 3 der Formeln

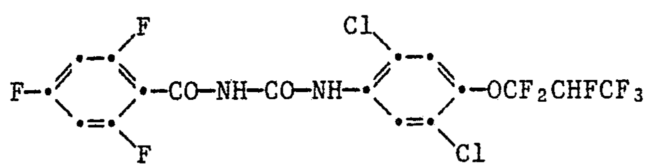 ,

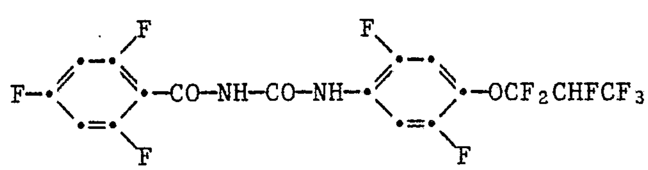 ,

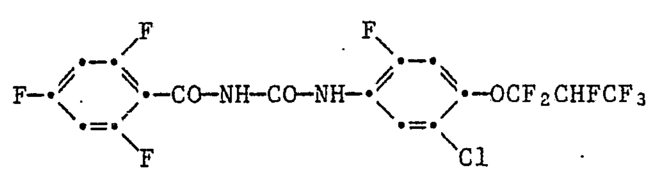 ,

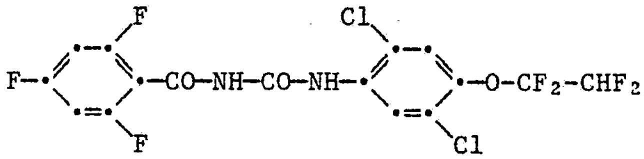 ,

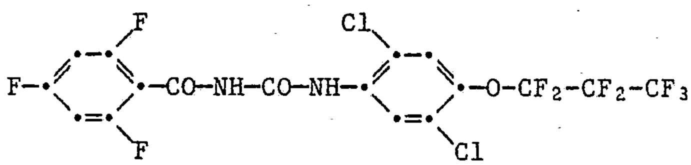 ,

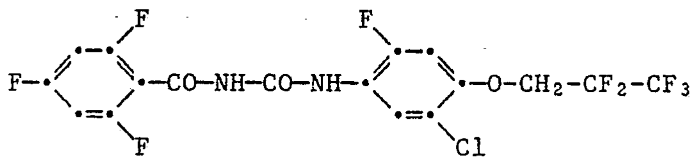 ,

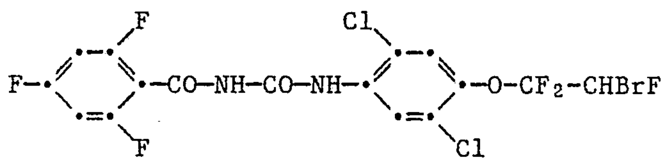 und

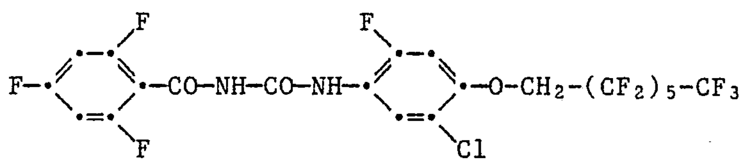 .

5. Verfahren zur Herstellung einer Verbindung der Formel I

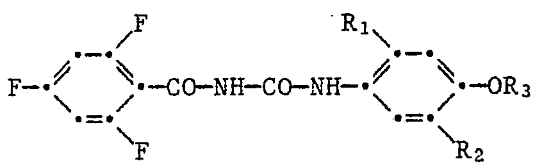 (I),

worin
$R_1$ für Fluor oder Chlor;
$R_2$ für Fluor, Chlor oder Brom; und
$R_3$ für ein- oder mehrfach durch Halogen substituiertes $C_1$-$C_7$-Alkyl
stehen, dadurch gekennzeichnet, dass man
a) ein Anilin der Formel II

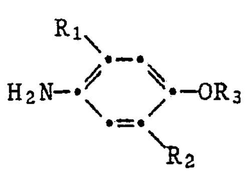 (II)

mit einem Benzoylisocyanat der Formel III

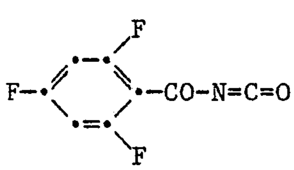 (III), oder

b) ein Isocyanat der Formel IV

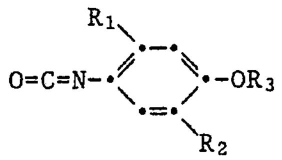

(IV)

mit einem Benzamid der Formel V

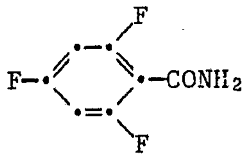

(V), oder

c) ein Anilin der Formel II mit einem Urethan der Formel VI

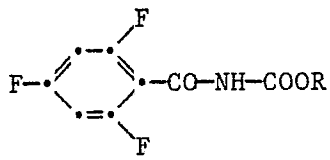

(VI)

angegebene Bedeutung haben und in Formel VI R für einen gegebenenfalls halogenierten $C_1$-$C_8$-Alkylrest steht.

6. Schädlingsbekämpfungsmittel, welches als aktive Komponente eine wirksame Menge einer Verbindung der Formel I

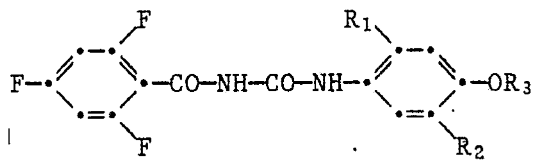

(I),

worin
$R_1$ für Fluor oder Chlor;
$R_2$ für Fluor, Chlor oder Brom; und
$R_3$ für ein- oder mehrfach durch Halogen substituiertes $C_1$-$C_7$-Alkyl
stehen, und geeignete feste oder flüssige Zuschlagsstoffe enthält.

7. Schädlingsbekämpfungsmittel gemäss Anspruch 6, welches als aktive Komponente eine Verbindung der Formel I enthält, worin $R_3$ für ein- oder mehrfach durch Fluor und/oder Chlor substituiertes $C_1$-$C_5$-Alkyl stehen.

8. Schädlingsbekämpfungsmittel gemäss Anspruch 7, welches als aktive Komponente eine Verbindung der Formel I enthält, worin $R_1$ und $R_2$ je für Fluor oder Chlor; und $R_3$ für ein- oder mehrfach durch Fluor substituiertes $C_1$-$C_4$-Alkyl stehen.

9. Verwendung einer Verbindung der Formel I

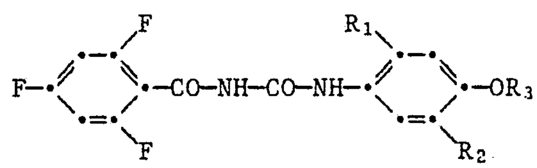

(I),

worin
$R_1$ für Fluor oder Chlor;
$R_2$ für Fluor, Chlor oder Brom; und
$R_3$ für ein- oder mehrfach durch Halogen substituiertes $C_1$-$C_7$-Alkyl
stehen, zur Kontrolle von Schädlingen an Tieren und Pflanzen.

10. Verwendung einer Verbindung der Formel I gemäss Anspruch 9 zur Kontrolle von Insekten und Arachniden.

11. Verwendung einer Verbindung der Formel I gemäss Anspruch 10 zur Kontrolle der larvalen Stadien von pflanzenschädigenden Insekten.

12. Verfahren zur Kontrolle von Schädlingen an Tieren und Pflanzen, dadurch gekennzeichnet, dass man die Schädlinge in ihren verschiedenen Entwicklungsstadien mit einer wirksamen Menge einer Verbindung der Formel I

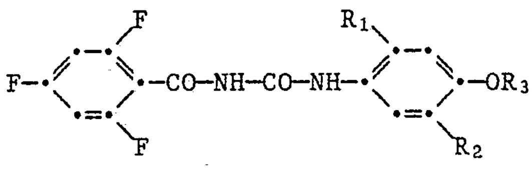

(I),

worin
$R_1$ für Fluor oder Chlor;
$R_2$ für Fluor, Chlor oder Brom; und
$R_3$ für ein- oder mehrfach durch Halogen substituiertes $C_1$-$C_7$-Alkyl stehen, in Kontakt bringt.

Europäisches Patentamt

# EUROPÄISCHER RECHERCHENBERICHT

Nummer der Anmeldung

EP 89 81 0346

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 232 080 (SUMITOMO CHEMICAL)<br>* Anspruch 1 *<br>--- | 1-12 | C 07 C 127/22<br>A 01 N 47/34 |
| Y | EP-A-0 235 089 (CIBA-GEIGY)<br>* Beispiel 1 *<br>--- | 1-12 | |
| Y | EP-A-0 246 061 (SUMITOMO CHEMICAL)<br>* Anspruch 1; Verbindungen 3,5,8,10 *<br>--- | 1-12 | |
| Y | EP-A-0 221 847 (CIBA-GEIGY)<br>* Anspruch 1 *<br>--- | 1-12 | |
| D,Y | US-A-4 518 804 (R.H. RIGTERINK et al.)<br>* Anspruch 1 *<br>----- | | |

RECHERCHIERTE SACHGEBIETE (Int. Cl.4)

C 07 C 127/00

Der vorliegende Recherchenbericht wurde für alle Patentansprüche erstellt

| Recherchenort | Abschlußdatum der Recherche | Prüfer |
|---|---|---|
| DEN HAAG | 10-08-1989 | WELLS A.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument